# EUROPEAN PATENT APPLICATION

(11) **EP 4 201 350 A1**
(43) Date of publication of application: **28.06.2023**
(21) Application number: 21217695.2
(22) Date of filing: 24.12.2021
(51) Int. Cl.: A61B 18/12, A61B 18/14, A61B 34/20, A61B 5/00, A61B 5/287, A61B 18/00

(54) **CONTROLLING AND MONITORING SIGNALS PROVIDED TO ELECTRODES**

(71) Applicant: Koninklijke Philips N.V., 5656 AG Eindhoven (NL)
(72) Inventor: KAHLMAN, Josephus Arnoldus Johannes Maria, Eindhoven (NL); MEGENS, Mischa, Eindhoven (NL)
(74) Representative: Philips Intellectual Property & Standards

(57) **Abstract**

An approach for providing and monitoring signals to/at electrodes of an interventional device system. An electrode controller defines the control signals provided to each active electrode connected to an electrode interface. A common current sensing system is used to monitor a reference signal through a reference electrode connector, connected to a reference electrode, to monitor a current from each active electrode to the reference electrode.

## Description

### FIELD OF THE INVENTION

The present invention relates to the field of interventional device systems, and in particular, to controlling and monitoring signals provided to electrodes of interventional device systems.

### BACKGROUND OF THE INVENTION

There is an increasing interest in interventional device systems. Interventional device systems comprise an interventional device and one or more electrodes, one or more of which are carried by the interventional device. An interventional device is a device that can be positioned within an anatomical cavity, in an anatomical structure, of an individual. There is a desire to control and/or monitor signals at any electrodes of the interventional device system, to achieve a variety of clinical tasks or goals.

One advantageous use case scenario is in the monitoring of the location of the interventional device. In such scenarios, the interventional device system can be configured to track the location of any electrodes carried by the interventional device, e.g., by monitoring the response of the electrode(s) to induced (crossing) electric fields.

One example of a suitable system that achieves this goal is the EPD Solutions navigation system provided by Philips^{®}, named the KODEX-EPD^{®} system. This system exploits electric field sensing for 3D localization, allowing radiation-free navigation in an anatomical cavity, e.g., inside the heart.

Some mapping techniques, such as those employed by the KODEX-EPD system, are based on electrical fields between three pairs of external electrodes that represent the x-, y-, and z-dimension. Voltages at electrodes carried by an interventional device (device electrodes) are sampled, and a real-time voltage-to-position function is formed. This function is continuously updated based on the measurements coming in. Once sufficiently updated, the voltage-to-position function enables the system to reliably determine and display the spatial movement of the catheter. If the interventional device is moved within an anatomical cavity or structure, it is possible to generate an anatomical model of the anatomical cavity or structure based on the monitored locations, as it can be assumed that the interventional device moves only within the cavity itself.

Other uses for controlling or monitoring signals provided to electrodes of an interventional device system would be apparent to the skilled person, e.g., to define a signal used for performing ablation using one or more electrodes of the interventional device or to determine electrophysical parameters (e.g., impedance, resistance or capacitance) of anatomical matter between electrodes.

There is therefore an increasing desire to improve the flexibility and reliability of controlling signals provided to electrodes of an interventional device system, as well as measuring such provided signals.

### SUMMARY OF THE INVENTION

The invention is defined by the claims.

According to examples in accordance with an aspect of the invention, there is provided a system for providing current, and monitoring provided current, to one or more active electrodes of an interventional device system.

The system comprises: an electrode interface comprising a plurality of electrode connectors each configured for connecting to a respective electrode of an interventional device system, the plurality of electrode connectors comprising at least: one or more active electrode connectors, each configured to connect to a respective active electrode at which an electrical signal is provided; a reference electrode connector configured to connect to a reference electrode; reference circuitry connected to the reference electrode connector, and configured to provide an electrical path from the reference electrode connector to a reference voltage; an electrode controller configured to provide, via the electrode interface, at least one control signal to each active electrode connected to the one or more active electrode connectors; and an electrode monitor comprising a common current sensing system, connected between the reference electrode connector and the reference voltage, configured to monitor a reference signal from the reference electrode connector to the reference voltage to thereby, when a reference electrode is connected to the reference electrode connector, monitor a magnitude of all currents flowing from any active electrode connected to the electrode interface to the reference electrode.

Embodiments are based on the realization that a single current sensing system, connected to a reference electrode, can be used to simultaneously monitor current passing through each active electrode to the reference electrode. Thus, rather than monitoring current at each active electrode, these currents can be monitored directly at the reference electrode. This approach provides an advantage of reduced circuitry and therefore reduced cost, material and power requirements. This approach also reduces differences between current measurements for different active electrodes that result from manufacturing intolerances and/or temperature drift of different individual current monitoring systems, providing a more robust and accurate monitoring system.

The approach also increases an accuracy of monitoring the current from an active electrode to the reference electrode. Monitoring this current directly at the active electrode would include any leakage currents at the active electrode in the monitored current, i.e., rather than just the current from the active electrode to the reference electrode. These leakage currents can be significant, as there is often additional circuitry coupled to the active electrode (connector), e.g., for filtering, safety, radiofrequency modulation and so on. In contrast, monitoring this current at the reference electrode avoids these leakage currents from contributing to a current measurement.

Embodiments thereby provide an efficient and accurate mechanism for monitoring current flow from active electrodes to a reference electrode.

In some examples, the electrode controller is configured such that at least one control signal provided to each of a subset of the active electrodes is of a different frequency to any other control signal (i.e. all other control signals) provided to any of the active electrodes; and the electrode monitor comprises a frequency demodulator configured to perform frequency demodulation of the reference signal to determine the contribution of each control signal, having a different frequency to any other control signal, to the reference signal.

This embodiment provides a mechanism for extracting the specific current flowing through each of a set of active electrodes, i.e., the contribution of each of the set of active electrodes to current flow through the reference electrode. Effectively, the control signals are modulated by frequency, so that frequency discrimination or filtering allows identifying of the contribution of each control signal. This approach provides a simple and intuitive mechanism for identifying current flow between specific active electrode(s) and the reference electrode. The subset may include all of the active electrodes or not all of the active electrodes.

In at least one example, the common current sensing system comprises: an impedance connected between the reference electrode connector and the reference voltage; and a voltage sensor configured to monitor a voltage across the impedance, the monitored voltage producing the reference signal. A current sense amplifier is an alternative option for the common current sensing circuit.

The system may further comprise a voltage sensing system configured to monitor a voltage of the control signal provided to each active electrode connected to the electrode interface.

In some examples, the voltage sensing system is configured to monitor a voltage between each active electrode connector and the reference electrode connector to thereby monitor the voltage of the control signal provided to each active electrode connected to the electrode interface.

The system may comprise an impedance system configured to process the voltages monitored by the voltage sensing system and the reference signal to thereby monitor an impedance between each active electrode and the reference electrode.

In some examples, the plurality of electrode connectors comprises at least two or more active electrode connectors; and the electrode controller is configured to operate in a differential mode in which: the electrode controller provides, via the electrode interface: a first control signal to a first active electrode connected to the electrode interface; a second control signal to a second active electrode connected to the electrode interface, wherein the first and second signals are of a same frequency and in antiphase with one another (i.e., of opposite polarity).

In some examples, the electrode controller further provides, via the electrode interface: a first calibration control signal to the first active electrode, the first calibration signal having a different frequency to any of the control signals.

The electrode controller may provide a second calibration control signal to the second active electrode, the second calibration signal having a different frequency to any of the control signals and the first calibration control signal.

In some examples, the frequency difference between the first control signal and the first calibration signal is less than 10% of the frequency of the first control signal. If present, the frequency difference between the second control signal and the second calibration signal is preferably less than 10% of the frequency of the second control signal.

The system may further comprise a balance measurement system configured to: filter the reference signal to extract a first portion, the first portion being the portion of the reference signal at the frequency of the first and second control signals; and determine a balance between the first control signal and the second control signal responsive to the extracted first portion.

The electrode controller may be configured to control the first control signal and/or second control signal responsive to the determined balance between the first control signal and the second control signal.

In some examples, the electrode controller is configured to control the at least one control signal provided to each active electrode responsive to the reference signal. In this way, the reference signal may act as feedback for defining or controlling the control signals provided to the active electrodes. In particular, the

The electrode controller may be configured to, for each active electrode connected to the electrode interface, control all control signals provided to the active electrode responsive to the determined contribution, to the reference signal, of each signal provided to the active electrode that has a different frequency to any other control signal (i.e. all other control signals).

Thus, if two or more control signals (e.g., a first control signal and a first calibration control signal) are provided to a same active electrode, then the contribution of a single one of these control signals could be used to control all of the two or more control signals. This facilitates feedback control over control signals that provide no or minimal contribution to the reference signal (e.g., due to the presence of another control signal of a same frequency but of opposite polarity).

The electrode interface may comprises: a first set of active electrode connectors, each configured to connect to an external active electrode for generating one or more electric fields; and a second set of active electrode connectors, each configured to connect to an internal active electrode for positioning within the one or more electric fields. The internal active electrode(s) may, for instance, be device electrodes carried by the interventional device.

In some examples, the electrode control system comprises, for each active electrode connector of the electrode interface, a respective synchronized circular waveform buffer for defining the control signal provided to any active electrode connected to the active electrode connector.

These and other aspects of the invention will be apparent from and elucidated with reference to the embodiments) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates an interventional device system in which embodiments can be implemented;
Figure 2 illustrates a system according to an embodiment;
Figure 3 illustrates a voltage control system for use in one embodiment;
Figure 4 illustrates a voltage control system for use in another embodiment; and
Figure 5 illustrates a method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an approach for providing and monitoring signals to/at electrodes of an interventional device system. An electrode controller defines the control signals provided to each active electrode connected to an electrode interface. A common current sensing system is used to monitor a reference signal through a reference electrode connector, connected to a reference electrode, to monitor a current from each active electrode to the reference electrode.

In the context of the present disclosure, a frequency of a signal is defined as the dominant frequency of the signal, i.e., the frequency between the largest peaks of the signal. A frequency of a signal is considered to be different to another signal if the dominant frequencies of the signals differ by a value detectable and distinguishable using conventional and existing frequency identification mechanisms, e.g., using lock-in amplifiers, FFT or DFT systems and the like.

It will be appreciated that, in the context of the present disclosure, a signal provided to an electrode is an electric signal and may represent a current, voltage and/or power provided to the electrode. Particular embodiments make use of current sources for providing current to an electrode, i.e. the signal is preferably a defined current.

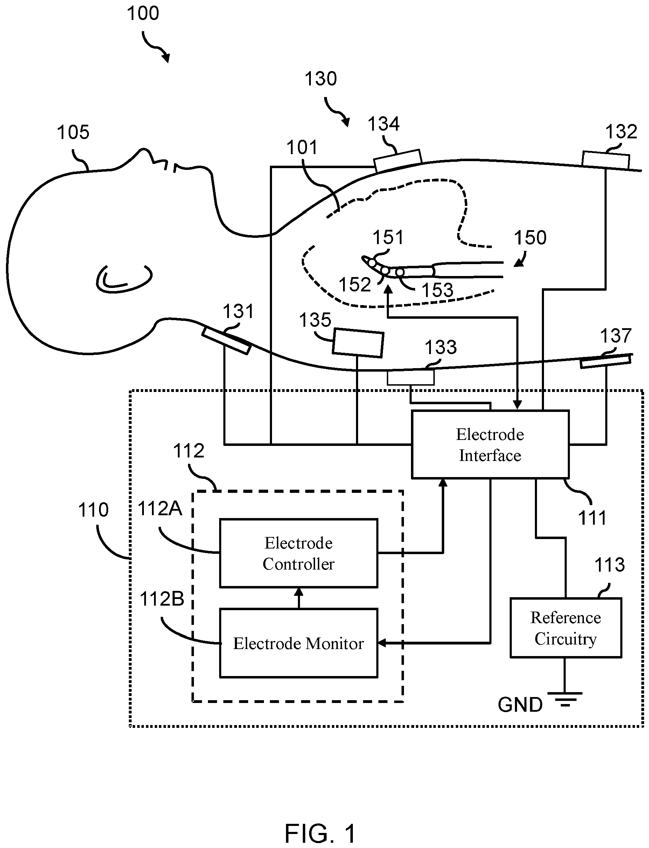

Figure 1 conceptually illustrates an interventional device system 100 in which embodiments can be employed.

The interventional device system 100 comprises an interventional device 150. The interventional device 150 is a device sized and configured to enter and move within an anatomical cavity 101 of a subject 105 or individual.

The interventional device system 100 also comprises a plurality of electrodes 131, 132, 133, 134, 135, 137, 151, 152, 153. Some of these electrodes, i.e., one or more of these electrodes, will act as active electrodes. An active electrode is one to which a current source is connected to provide positive or negative current thereto. In this way, a current is provided to flow to/from/through the active electrode. One of the plurality of electrodes will act as a reference electrode 137. A reference electrode provides a path to ground for any current injected (e.g., into the body) by an active electrode, i.e., it acts as a current drain. The reference electrode may be positioned at a predetermined position on the subject, e.g., on the right leg.

Different types of active electrodes are envisaged, including external active electrodes 131, 132, 133, 134, 135 for positioning on the surface of the subject 105 and internal/device active electrodes 151, 152, 153 carried by the interventional device 150.

The illustrated electrodes are purely exemplary, and any combination or number of electrodes may be implemented according to various embodiments of the interventional device system.

The interventional device system 100 also comprises a system 110 configured to provide current, and monitor provided current, to any active electrode(s) of the interventional device system. The system 110 may itself form an embodiment according to various examples.

The system 110 comprises an electrode interface 111. The electrode interface 111 comprises a plurality of electrode connectors, each configured to connect to a respective electrode of the interventional device system 100. Each electrode connector may, for instance, connect to a conductive lead/wire passing to an electrode.

The electrode connectors includes one or more active electrode connectors. Any electrode connected to an active electrode connector is defined as an active electrode. The electrode connectors also includes a reference electrode connector. The electrode connected to the reference electrode connector is defined as a/the reference electrode.

The system 110 also comprises an electrode control system 112.

The electrode control system comprises an electrode controller 112A, communicatively coupled to the electrode interface. The electrode controller is configured to provide at least one control signal to each active electrode (via the appropriate active electrode connector(s)). Each control signal defines a current flow to/from/through the active electrode to which it is connected. The electrode controller 112A provides this control signal via the electrode interface 111.

The electrode control system 112 also comprises an electrode monitor 112B. The electrode monitor 112B is configured to monitor one or more electrical parameters associated with each electrode, such as a current flowing through each electrode and/or a voltage at each electrode. This information could be used, for example, as a feedback for the electrode controller 112A of the electrode control system 112 and/or to derive information about the subject 105 and/or interventional device 150. Thus, the electrode monitor 112B may provide feedback or a feedback signal to the electrode controller.

The electrode controller 112A and/or electrode monitor may operate using a microprocessor, one or more analogue-to-digital converters and/or one or more digital-to-analogue converters. Other suitable examples for alterative systems that can receive electrical, process the receive signals and output signals will be readily apparent to the skilled person. The electrode controller 112A and electrode monitor 112B may represent two modules of a processing unit, i.e. the electrode control system 112.

One example use for the electrodes of the interventional device system 100 is as an ablation system. The one or more active electrodes may comprise an ablation electrode 151 carried by the interventional device 150. The electrode control system 112 may supply a current to the ablation electrode 151, via the electrode interface 111, to control or perform ablation on the subject (e.g. responsive to a user input signal or the like). The electrode monitor 112B may be used to monitor the current and/or power provided to the ablation electrode, to ensure ablation is performed correctly.

Another example use for the electrodes of the interventional device system 100 is for a device tracking system. The one or more active electrodes may comprise external electrodes positioned externally to the subject. Power, i.e., current, provided to the external electrodes may be configured to generate crossing electric fields induced within the subject. The electrical response of one or more device electrodes 151, 152, 153 carried by the interventional device 150 can be used to track the relative location of the interventional device within the crossing electric fields.

More specifically, signals detected/sensed by the device electrodes 151, 152, 153 can be used to track the location of each device electrode and therefore the interventional device 150. In particular, the electrical response of each device electrode of the interventional device 150 to crossing electric fields induced within the subject can be monitored and mapped to a location in a predefined co-ordinate system (sometimes called an R-space) using an appropriate mapping function. This mapping function is used at least partly because induced electrical fields' distribution is inherently inhomogeneous due to the different dielectric properties and field absorption (related to conductivity) of the interrogated tissues.

Approaches for generating a mapping function are well established in the art. Example processes are disclosed by the European Patent Applications EP 0775466 A2, EP 3568068 A1 and EP 3607879 A1. Typically, generating a mapping function makes use of known interelectrode distances to calibrate for the inhomogeneity in the crossing electric fields.

The tracked locations (e.g., in the predefined co-ordinate system) may be used to construct an anatomical model of the anatomical cavity in which the interventional device 150 travels. This is based on the assumption that the interventional device 150 will not be able to extend outside the bounds of the anatomical cavity 101, so that points of a tracked location represent points inside the anatomical cavity. A cloud of points can be reconstructed into an anatomical model using any known system, such as those discussed in Berger, Matthew, et al. "A survey of surface reconstruction from point clouds." Computer Graphics Forum. Vol. 26. No. 1. 2017. Further mechanisms will be readily apparent to the skilled person.

Yet another example use for the electrodes of the interventional device system 100 is to determine electrophysical parameters of the subject. For instance, an impedance between two electrodes may be determined to identify an impedance of part of the subject's tissue. As another example, the electrical response of electrodes to electrical activity of the subject may be monitored, e.g., to determine internal cardiac activation voltages or the like.

Yet another example use for the electrodes of the interventional device system 100 is to monitor an interaction between the interventional device 150 and the subject 105. In particular, an impedance between one of the device electrodes and another electrode (e.g., the reference electrode or an external electrode) will change responsive to a contact force between the device electrode and tissue of a subject. This is because the pressure pushes the device electrode into the tissue, which effectively changes the proportion of tissue effects in relation to the blood pool.

As yet another example, external electrodes may be controlled and monitored for performing an electrical impedance tomography (EIT) imaging technique. EIT is a well-known technique (e.g., for measuring fluid in lungs) in which the response of currents between external electrodes is detected and processed into an image.

A number of other uses for electrodes of an interventional device system 100 are well known in the art.

The system also provides reference circuitry 113. The reference circuitry provides an electrical path from the reference electrode connector to a reference voltage (e.g., a ground voltage GND, although other non-ground voltages may act as the reference voltage). In this way, the reference circuity provides an electrical path from a reference electrode connected to the reference electrode connector to the reference voltage GND.

The reference voltage may be an earth or a ground voltage GND. However, in some circumstances, it may be advantageous to provide a non-earthed reference voltage. For instance, the electrodes may be used to measure internal cardiac activation voltages, which are ECG-like signals. For this purpose, the reference electrode may be driven to force the Wilson voltage to zero volt to ensure proper scaling and/or offsetting.

Approaches proposed in this disclosure provide an improved electrode monitor 112B, which is able to more accurately detect at least a current flowing from/to/through each active electrode. This can improve a measurement of one or more electrical parameters and/or provide improved feedback for controlling the operation of the electrode controller.

Some embodiments also provide an improved electrode controller 112A, which provides more flexible control over the current suppled to each active electrode, e.g., each electrode connected to the one or more active electrode connectors.

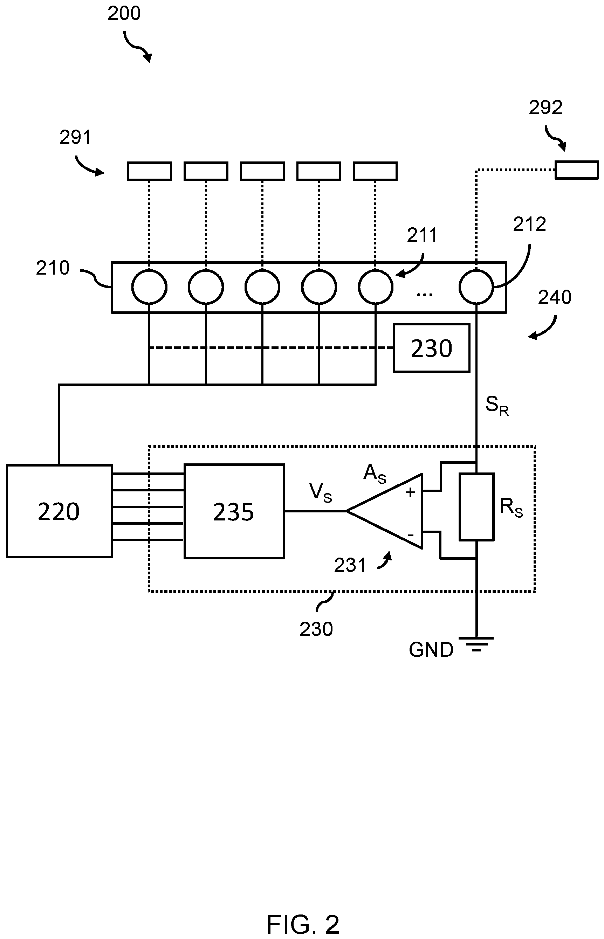

Figure 2 illustrates a portion of a system 200 according to an embodiment.

The system 200 comprises an electrode interface 210, an electrode controller 220, an electrode monitor 230 and reference circuitry 240.

The electrode interface 210 comprises one or more active electrode connectors 211 (here: a plurality of active electrode connectors). Each active electrode connector is configured to (electrically) connect or be connectable to a respective active electrode 291. This connection may be via a lead or wire, illustrated using a dotted line.

The electrode controller 220 is configured to provide at least one control signal to each active electrode connected to the active electrode connector(s). In particular, in the hereafter described example, the electrode controller 220 is configured to control a current (e.g., an amplitude, frequency and phase of the current) provided to each active electrode connected to the active electrode connector(s).

For the sake of explanation, only five active electrode connectors (and corresponding active electrodes) are illustrated. However, any number N of active electrode connectors (with a corresponding number of active electrodes) may be provided, where N is any real positive integer. For instance, N may be greater than 10, e.g., greater than 50, e.g., greater than 100.

The electrode interface 210 also comprises a reference electrode connector 212, which is configured to (electrically) connect or be connectable to a reference electrode 292. This connection may be via a lead or wire, illustrated using a dotted line. The reference circuitry 240 provides a path to a reference voltage GND (e.g., ground) for electrical signals received at the reference electrode connector, and therefore any electrical signals received at a reference electrode 292 connected thereto.

The electrode monitor 230 comprises a common current sensing system 231. The common current sensing system 231 is configured to monitor a reference signal S_{R} from the reference electrode connector 212 to the reference voltage GND. This thereby monitors a current flowing from any of the active electrodes to the reference electrode.

In the illustrated example, the common current sensing system 231 comprises an impedance R_{S} (e.g., a resistor) connected between the reference electrode connector 212 and the reference voltage GND; and a voltage sensor A_{S} configured to monitor a voltage Vs across the impedance, the monitored voltage producing or representing the reference signal (in voltage form). Here, the voltage sensor A_{S} is a differential amplifier coupled across the impedance R_{S}, but other forms of voltage sensors would be apparent to the skilled person. The illustrated common current sensing system 231 thereby provides a voltage Vs that changes responsive to the current of the reference signal S_{R}, such that the voltage Vs contains information responsive to a magnitude of all currents flowing from any active electrode connected to the electrode interface to the reference electrode.

The common current sensing system 231 may instead be replaced with a current sense amplifier, as one alternative. Other approaches for monitoring a current between two nodes (the reference electrode connector 212 and the reference voltage GND) would be apparent to the skilled person, and could be implemented in alternative embodiments.

The use of the common current sensing system 231 (communicatively coupled to the reference electrode 292) has the effect of more efficiently and accurately monitoring a true current induced in the body by each active electrode, e.g. between each active electrode and the reference electrode.

By way of explanation, it is herein recognized that the provision of a well-defined and stable current to an active electrode by the electrode controller 220 is a difficult task, and existing hardware often requires additional circuitry (i.e., beyond a direct connection to a current supply) to provide a safe and desired current (e.g., protection and filtering circuitry, frequency generators and so on).

There may be other reasons for connecting additional circuitry to an active electrode. One other example of additional circuitry connected to an active electrode might include a logging device, e.g., for logging and recording subject-generated electrical signals, such as activation signals of the heart. Another example of additional circuitry that may be connected to an active electrode is a pacer, e.g., for injecting current into the heart tissue for triggering a response.

It has been recognized that this additional circuitry introduces parasitic impedances, e.g., causing one or more leakage currents. Thus, monitoring a current output by the current source for provision to an active electrode at the active electrode itself (or the circuitry connected to the active electrode) would not accurately reflect the true current between the active electrode and the reference electrode, which is of most relevance for accurately providing/monitoring electrical signals to the individual. This is because any such measurement of current, at the active electrode, would also include any leakage currents - which do not contribute to the true current induced in the body by the active electrode.

In other words, there are leakage currents in analog circuits and parasitic resistances that would contribute to a current measurement at the active electrode connector, but would not contribute to the actual current between the active electrode and the reference electrode inside the body. Use of the common current sensing system thereby allows these leakage currents to be automatically discounted from the measurement of the current between electrodes.

The use of a common current sensing system 231 also avoids the need for individual current sensing systems for each active electrode. This can significantly reduce an amount of circuitry required to monitor currents, thereby reducing complexity, size and power consumption of the system. The common current sensing system also facilitates improved accuracy, because use of a single current sensing system (rather than a current sensing system for each active electrode) avoids differences resulting from manufacturing tolerances and temperature drift.

The electrode controller 220 may be configured to regulate the control signals provided to the active electrode connector(s) responsive to the monitored reference signal, e.g., the voltage Vs. In this way, the reference signal can act as feedback for the control of the control signals provided to the active electrode connector(s).

In a preferred examples, the electrode controller 220 is configured such that at least one control signal provided to each of a subset of the active electrodes is of a different frequency to any other control signal provided to any of the active electrodes.

This approach means that the current flowing from any of the subset of active electrodes to the reference electrode will be distinguishable or discriminable in the frequency domain. Thus, it is possible to filter out other frequencies in the reference signal S_{R} (or the voltage Vs) to measure/monitor the specific current flowing from each of the subset of active electrodes to the reference electrode.

Thus, the electrode monitor may comprise a frequency demodulator 235 configured to perform frequency demodulation of the reference signal to determine the contribution of each control signal, having a different frequency to any other control signal, to the reference signal. This facilitates identification of the individual current contribution from each of the subset of active electrodes. The demodulation is schematically illustrated in Figure 2.

The frequency demodulator is a device that performs frequency filtering on a signal to identify sub-signals lying in different frequency bands. Each frequency band contains or corresponds to a different possible frequency for a control signal to be provided to an active electrode. The frequency demodulator may operate in the digital domain (e.g., using a Fourier transform technique, such as FFT or DFT) or in the analogue domain (e.g., using dedicated analog filters for separating different frequency components).

This approach effectively treats the reference signal as a frequency multiplexed group of signals, i.e., the reference signal is treated as carrying a plurality of channels - each channel representing a different control signal of different frequencies (or, as later described, pair of control signals).

Thus, the frequency demodulator 235 is able to discriminate between different frequencies, to thereby identify portions of the reference signal that represent any frequency-specific control signal provided to any of the active electrodes. This provides an approach for separating out the contributions from each active electrode, to thereby determine and monitor a measure of a current passing through each active electrode.

Other approaches could be adopted, e.g. the control signals could be provided using a time multiplexing approach, and a time de-multiplexing mechanism could be used to discriminate or identify which control signal is represented at a point in time for the reference signal.

The identified portions could be used by the electrode controller 220 as feedback for controlling or modifying control signals provided to each active electrode connected to the electrode interface.

The subset may comprise all of the active electrodes or not all of the active electrodes. This may depend upon a mode of operation in which the electrode controller controls each active electrode.

The electrode controller 220 may be configured to selectively control each active electrode (connected to the active electrode connector(s)) in a single-ended mode or a differential mode. The mode of operation does not need to be the same for all active electrodes (or each pair of active electrodes).

Controlling an active electrode in a single-ended mode results in a control signal being provided to that active electrode, wherein the frequency of the control signal is different to all other control signals provided to any (i.e. all) other active electrode.

Controlling an active electrode in a differential mode results in a control signal being provided to that active electrode in which the frequency of the control signal is the same as one other control signal provided to another other active electrode, but in anti-phase with (i.e., of opposite polarity to) that other control signal.

It will be appreciated that, for the differential mode, each active electrode being controlled in the differential mode will typically form one of a pair of active electrodes being controlled in the differential mode. Thus, there may be an even number of active electrodes being controlled in the differential mode.

For active electrodes that are controlled in the single-ended mode, the part of the reference signal S_{R} having the same frequency as the control signal suppled to said active electrode directly represents a current flowing from the active electrode to the reference electrode. This facilitates direct monitoring of this current using the electrode monitor 230, which could be used to provide direct feedback on the current (e.g., to achieve a desired current).

For active electrodes that are controlled in the differential mode, the part of the reference signal S_{R} having the same frequency as the control signal suppled to said active electrode directly represents an imbalance between the current supplied to the active electrode by the electrode controller and the current supplied to the other active electrode to which a control signal of the same frequency, but opposite polarity, is provided. Thus, direct feedback on the magnitude of the current for the control signal supplied to an active electrode (at the frequency shared by another control signal) is not possible. However, information about the imbalance would still remain useful for controlling the control signals provided to each active electrode in the pair, e.g., to reduce an imbalance.

Thus, the system may comprise a balance measurement system configured to: filter the reference signal to extract a first portion, the first portion being the portion of the reference signal at the frequency of the first and second control signals; and determine a balance between the first control signal and the second control signal responsive to the extracted first portion.

A balance or imbalance represents a difference in magnitude between the current supplied by the two active electrodes in the pair of active electrodes (associated with the same control signal frequency).

To obtain information about an active electrode controlled in the differential mode, the electrode controller may be configured to provide a supplementary or calibration control signal to such an active electrode. This calibration control signal is in addition to the control signal having a same frequency, but opposite polarity, to a control signal supplied to another active electrode - which could be labelled the "main control signal". The calibration control signal may have a frequency that is different to all other control signals provided to any other active electrode, including any other calibration control signal (i.e. all other calibration control signals).

The calibration control signal will undergo similar leakage and/or attenuation to the main control signal, as it would be processed by the same additional circuitry (if present). Thus, if the electrode monitor is able to monitor the calibration control signal (e.g., through frequency discrimination), then it is possible to effectively monitor the main control signal. Thus, the calibration control signal can act as a substitute or proxy for the main control signal.

If a calibration control signal is suppled to each active electrode in the pair of active electrodes (which are supplied with a control signal of a same frequency but opposite polarity), then it is possible to determine information about the difference between the main control signals provided to the pair of active electrodes, i.e., the differential signal provided by the pair.

Alternatively, in some examples, only one calibration control signal is used for a pair of active electrodes operating in the differential mode. In this case the amplitude of the differential current could be calculated from the measured unbalance (measured at the frequency of the main control signals provided to each active electrode) and the monitored calibration control signal (measured at the frequency of the calibration control signal provided to one of the pair of active electrodes).

Preferably, the calibration control signal has a frequency close to the frequency of the main control signal provided to the same active electrode, e.g., within 10% of the main control signal or within 5% of the main control signal. This means that the calibration control signal will respond in a similar manner to the control signal provided to the same active electrode, e.g., exhibit similar leakage and/or attenuation, should such effects be frequency dependent. This improves an accuracy in determining information about the control signal.

It may be advantageous to apply more than one calibration control signal (at a respective more than one frequency) to any active electrodes operating in the differential model. This may facilitate determination of frequency dependencies and parasitic effects more accurately.

For instance, for a particular active electrode a first calibration control signal may be applied at a frequency M% greater than the main control signal and a second calibration control signal may be applied a frequency M% less than the main control signal. M is any positive value, e.g. 5 or 10. The electrode monitor may monitor the current of the calibration control signals by monitoring the reference signal, e.g. using any previously described approach. The determined currents may then be averaged to generate a predicted current for the main control signal. This approach provides a mechanism for estimating the current of the main control signal, whilst mitigating any difference due to frequency dependency.

Any calibration control signals may have a different amplitude to the control signal provided to the same active electrode, e.g., a fraction of the main control signal. This can be compensated in any calculations.

Of course, calibration control signals could also be provided to any active electrode controlled in the single-ended mode. In this context, the main control signal may be the control signal with a greatest amplitude.

The electrode controller 220 may, for instance, comprise a single-ended current driver for each active electrode controller. Each single-ended current driver may be driven by a synchronized circular waveform buffer. Each circular waveform buffer may be formed of a circular buffer of N-stages, so that the buffer can generate frequencies at multiples of fs^{∗} 2/N This approach facilitates the generation of a range of currents with different frequencies, phases and amplitudes and a variety of simultaneous single-ended and differential modes.

This approach facilitates the provision of more than one control signal to each electrode, as well as control over the amplitude (e.g., in Amperes) of the control signal provided to each electrode (which has not previously been available).

The electrode controller 220 may further comprise, for each active electrode, one or more additional circuitry elements. These additional circuitry elements may comprise protection and/or filtering circuits and/or radiofrequency generators (e.g., for performing ablation). Other examples include loggers and/or pacers, which have been previously described.

The electrode controller 220 may comprise an analogue to digital converter, e.g., for converting the voltage(s) provided by the electrode monitor 230 to a digital form. The electrode controller may use the converted voltage(s) to determine a control strategy for the active electrode(s). The electrode controller 220 may then control the operation of a current driver, e.g., for each active electrode connector, responsive to the determined control strategy to control the current supplied to each active electrode. This control may make use of a digital to analogue converter.

One potentially valuable electrical parameter for an interventional device system is an impedance, i.e., an impedance between two electrodes. This may be an impedance between an active electrode and a reference electrode, or between two active electrodes.

An impedance can be calculated/approximated by dividing the voltage difference between the two electrodes by the current between the two electrodes. The current between the two electrodes is determined using the monitored reference signal, as previously described.

Thus, to calculate an impedance, the voltage at each electrode should be determined. Thus, the system 200 may further comprise a voltage sensing system 250 configured to monitor a voltage of the control signal provided to each active electrode connected to the electrode interface.

The voltage at the reference terminal is represented by the sense voltage Vs.

The voltage sensing system may be configured to monitor a voltage between each active electrode connector and the reference electrode connector to thereby monitor the voltage of the control signal provided to each active electrode connected to the electrode interface.

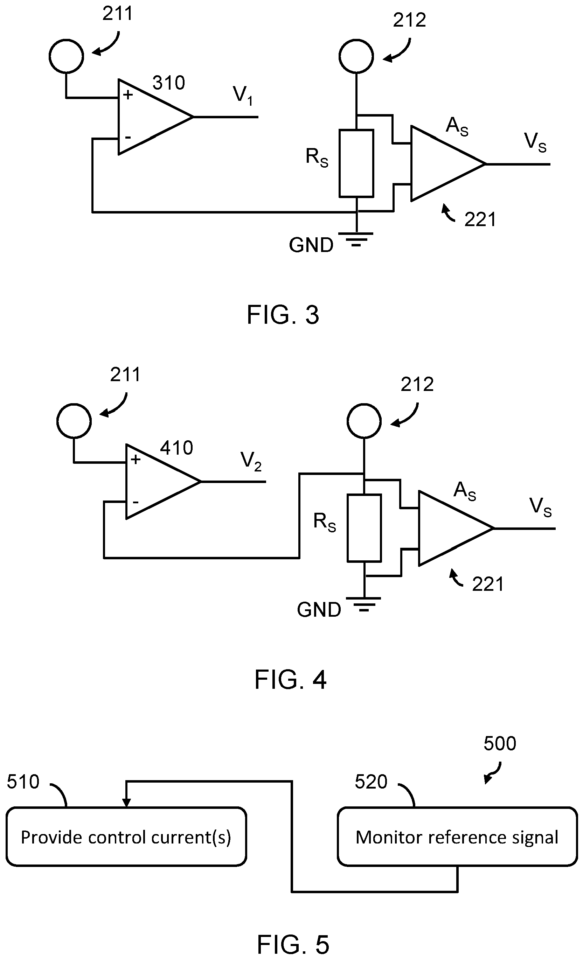

Figure 3 illustrates a first approach for monitoring a voltage between an active electrode connector 211 and the reference electrode connector 212.

In this example, a voltage sensor 310 is configured to monitor a first voltage V₁, being a difference in the voltage at the active electrode connector 211 and the ground or reference voltage GND. The voltage across the impedance R_{S} of the common current sensing system is then subtracted from the first voltage (either in the analogue domain: as illustrated, or in a digital domain) to produce the voltage of the control signal provided to the active electrode.

Figure 4 illustrates an improved approach for monitoring a voltage between an active electrode connector and the reference electrode connector

In this example, a voltage sensor 410 is configured to monitor a second voltage V₂, being a difference in the voltage at the active electrode connector 211 and the voltage at the reference electrode connector 212. This avoids the need for a subtraction, saving processing power and/or circuitry.

Either of these voltage sensors could be implemented into the voltage sensing system 250 illustrated in Figure 2. Each voltage sensor may, for instance, be a high-impedance low-noise amplifier.

Figure 5 illustrates a method 500 according to an embodiment.

The method 500 is configured for controlling a system that provides current, and monitors provided current, to one or more active electrodes of an interventional device system, the system comprising: an electrode interface comprising a plurality of electrode connectors each configured for connecting to a respective electrode of an interventional device system, the plurality of electrode connectors comprising at least: one or more active electrode connectors, each configured to connect to a respective active electrode at which an electrical signal is provided; a reference electrode connector configured to connect to a reference electrode.

The method comprises a step 510 of using an electrode controller to provide, via the electrode interface, at least one control signal to each active electrode connected to the one or more active electrode connectors.

The method also comprise a step 520 of using an electrode monitor comprising a common current sensing system to monitor a reference signal from the reference electrode connector to the reference voltage. The common current sensing system is connected between the reference electrode connector and the reference voltage. Step 520 thereby, when a reference electrode is connected to the reference electrode connector, monitors a magnitude of all currents flowing from any active electrode connected to the electrode interface to the reference electrode.

The output of step 520 may be used to perform step 510. In other words, the monitored magnitude of all currents flowing from any active electrode connected to the electrode interface to the reference electrode may be used to define or control the control signal(s).

In any described embodiment of the invention, the electrode controller and/or the electrode monitor may operate as or comprise a processing system. A processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. A system (200) for providing current, and monitoring provided current, to one or more active electrodes (131, 132, 133, 134, 135, 137, 151, 152, 153, 291) of an interventional device system (100), the system comprising:
an electrode interface (111, 210) comprising a plurality of electrode connectors each configured for connecting to a respective electrode of an interventional device system, the plurality of electrode connectors comprising at least:
one or more active electrode connectors (211), each configured to connect to a respective active electrode (291) at which an electrical signal is provided;
a reference electrode connector (212) configured to connect to a reference electrode (292);
reference circuitry (113, R_{S}) connected to the reference electrode connector, and configured to provide an electrical path from the reference electrode connector to a reference voltage (GND);
an electrode controller (112A, 220) configured to provide, via the electrode interface, at least one control signal to each active electrode connected to the one or more active electrode connectors; and
an electrode monitor (112B, 230) comprising a common current sensing system, (231) connected between the reference electrode connector and the reference voltage, configured to monitor a reference signal from the reference electrode connector to the reference voltage to thereby, when a reference electrode is connected to the reference electrode connector, monitor a magnitude of all currents flowing from any active electrode connected to the electrode interface to the reference electrode.

2. The system of claim 1, wherein:
the electrode controller is configured such that at least one control signal provided to each of a subset of the active electrodes is of a different frequency to any other control signal provided to any of the active electrodes; and
the electrode monitor comprises a frequency demodulator configured to perform frequency demodulation of the reference signal to determine the contribution of each control signal, having a different frequency to any other control signal, to the reference signal.

3. The system of claim 1 or 2, wherein the common current sensing system comprises:
an impedance connected between the reference electrode connector and the reference voltage; and
a voltage sensor configured to monitor a voltage across the impedance, the monitored voltage producing the reference signal.

4. The system of any of claims 1 to 3, further comprising a voltage sensing system configured to monitor a voltage of the control signal provided to each active electrode connected to the electrode interface.

5. The system of claim 4, wherein the voltage sensing system is configured to monitor a voltage between each active electrode connector and the reference electrode connector to thereby monitor the voltage of the control signal provided to each active electrode connected to the electrode interface.

6. The system of claim 4 or 5, further comprising an impedance system configured to process the voltages monitored by the voltage sensing system and the reference signal to thereby monitor an impedance between each active electrode and the reference electrode.

7. The system of any of claims 1 to 6, wherein:
the plurality of electrode connectors comprises at least two or more active electrode connectors; and
the electrode controller is configured to operate in a differential mode in which:
the electrode controller provides, via the electrode interface:
a first control signal to a first active electrode connected to the electrode interface;
a second control signal to a second active electrode connected to the electrode interface, wherein the first and second signals are of a same frequency and in antiphase with one another.

8. The system of claim 7, wherein the electrode controller further provides, via the electrode interface:
a first calibration control signal to the first active electrode, the first calibration signal having a different frequency to any of the control signals.

9. The system of claim 8, wherein:
the frequency difference between the first control signal and the first calibration signal is less than 10% of the frequency of the first control signal.

10. The system of any of claims 7 to 9, further comprising a balance measurement system configured to:
filter the reference signal to extract a first portion, the first portion being the portion of the reference signal at the frequency of the first and second control signals;
determine a balance between the first control signal and the second control signal responsive to the extracted first portion.

11. The system of claim 10, wherein the electrode controller is configured to control the first control signal and/or second control signal responsive to the determined balance between the first control signal and the second control signal.

12. The system of any of claims 1 to 11, wherein the electrode controller is configured to control the at least one control signal provided to each active electrode responsive to the reference signal.

13. The system of claim 12, when dependent upon claim 2, wherein the electrode controller is configured to, for each active electrode connected to the electrode interface, control all control signals provided to the active electrode responsive to the determined contribution, to the reference signal, of each signal provided to the active electrode that has a different frequency to any other control signal.

14. The system of any of claims 1 to 13, wherein the electrode interface comprises:
a first set of active electrode connectors, each configured to connect to an external active electrode for generating one or more electric fields; and
a second set of active electrode connectors, each configured to connect to an internal active electrode for positioning within the one or more electric fields;

15. The system of any of claims 1 to 14, wherein the electrode control system comprises, for each active electrode connector of the electrode interface, a respective synchronized circular waveform buffer for defining the control signal provided to any active electrode connected to the active electrode connector.
